# EUROPEAN PATENT APPLICATION

(11) **EP 2 258 364 A1**
(43) Date of publication of application: **08.12.2010**
(21) Application number: 09728612.4
(22) Date of filing: 31.03.2009
(51) Int. Cl.: A61K 31/4365, A61K 31/4196, A61P 7/02, A61P 9/00, A61P 9/08, A61P 9/10, A61P 43/00

(54) **PREVENTIVE AND/OR REMEDY FOR VASCULAR DISEASES**

(30) Priority: 01.04.2008 JP 2008095370
(71) Applicant: Astellas Pharma Inc., Tokyo 103-8411 (JP)
(72) Inventor: KAWASAKI, Tomihisa, Tokyo 103-8411 (JP); FUNATSU, Toshiyuki, Tokyo 103-8411 (JP); SAKURAI, Chinatsu, Tokyo 103-8411 (JP)
(74) Representative: Gille Hrabal Struck Neidlein Prop Roos
(86) International application number: PCT/JP2009/056708
(87) International publication number: WO 2009/123210

(57) **Abstract**

[Object] To provide an excellent pharmaceutical composition for preventing and/or treating vascular diseases.

[Means for Solution] Useful to provide a pharmaceutical composition for preventing and/or treating vascular diseases, which comprises 1) a COX-1 selective inhibitor and 2) clopidogrel or a pharmaceutically acceptable salt thereof. The present invention is useful as an excellent pharmaceutical composition for preventing and/or treating vascular diseases is provided and is particularly useful as a pharmaceutical composition for preventing and/or treating arterial thrombosis, ischemic heart disease, ischemic brain disease, pulmonary embolism, peripheral circulation disorder, restenosis and reocclusion, essential thrombocytosis and so on is provided.

## Description

### TECHNICAL FIELD

This invention relates to an agent for preventing and/or treating vascular diseases, **characterized in that** a COX-1 selective inhibitor or a pharmaceutically acceptable salt thereof is combined with clopidogrel or a pharmaceutically acceptable salt thereof.

### BACKGROUND OF THE INVENTION

Since its discovery by Donne in 1942 (C. R. Acad. Sci. (Paris), 14, 336 - 68, 1842), platelet has been treated for a long time as a blood component which is necessary for hemostasis. In these days, it has been revealed that platelet not only merely plays the main role of hemostasis mechanism but also shows multifunctional properties such as clinically noteworthy arteriosclerosis formation, circulatory organ diseases including thrombotic diseases, cancer metastasis, inflammation, rejection reaction after transplantation and participation in immune reaction.

In recent years, PTCA therapy and stent placement method have been rapidly spreading and getting certain results for the treatment of coronary stenosis- and aortic stenosis-based diseases such as angina pectoris, myocardial infarction and the like. However, these therapeutic methods injure blood vessel tissues including endothelial cells, thus posing a problem of causing acute coronary occlusion and, further, restenosis which occurs at the chronic stage. Platelet exerts an important role in various thrombotic events after such revascularization therapies (Catheter Cardiovasc. Interv., 69: 637 - 42, 2007). Accordingly, efficacy of an anti-platelet agent is desired, but sufficient effect by the conventional anti-platelet agents has not been proved yet.

Under such circumstances, anti-platelet agents such as aspirin, cilostazol, prostaglandin I₂, prostaglandin E₁, ticlopidine, clopidogrel (Patent References 2 and 3), dipyridamole and the like have been used as preventive or therapeutic agents for these circulatory organ system diseases. Among these drugs, aspirin and clopidogrel are now generally used alone or concomitantly, with the aim of carrying out secondary prevention of the thrombotic event of thromboembolism patients.

Though clopidogrel significantly lowered the event onset ratio by a factor of 8.7% based on aspirin (clopidogrel 5.83%/year vs. aspirin 5.32%/year) in a CAPRIE trial carried out using thromboembolism patients as the subjects, the difference is not considerable (Non-patent Reference 1), so that a demand has been directed toward the appearance of a drug which shows higher event inhibition ratio.

On the other hand, a result of meta-analysis has been reported on the event inhibition ratio of aspirin, which was 19% by its administration of from 500 to 1500 mg, 26% by its administration of from 160 to 325 mg, 32% by its administration of from 75 to 150 mg and 13% by 75 mg or less, so that clear dose-dependency was not found (Br. J. Med., 324: 71 - 86, 2002). In addition, a result of meta-analysis has also been reported on gastrointestinal bleeding as a side effect of aspirin, but dose-dependency was not found on its expression frequency and the frequency was from 2 to 3% (Br. J. Med., 321: 1183 - 7, 2000). Based on these analytical results, low dose aspirin has been recommended when prevention of thrombotic event is the object, but particularly, it has been reported that the event inhibition ratio for high risk patients was low, namely about 25% (N. Engl. J. Med., 353: 2373 - 83, 2005).

As described in the above, the event inhibition ratio of aspirin and clopidogrel is not satisfactory, and it is considered to be insufficient particularly for the high risk patients of acute coronary syndrome (ACS) and the like. Based on such a background, combination use effect of clopidogrel on aspirin has been examined in a CURE trial on non-ST increase ACS patients as the objects. According to this, the event ratio was significantly reduced by a factor of 20% in the combination use group, in comparison with the aspirin alone group (Non-patent Reference 2). In addition, there has been reported a significant secondary thrombotic event inhibition effect at the time of combination use of aspirin and clopidogrel for the patients of ischemic diseases, also by a CHARISMA trial (Non-patent Reference 3). However, it has been reported that hemorrhagic side effects were significantly increased in the combination use group by the CURE trial, and usefulness of the combination group was not found regarding primary prevention effect of low risk patients by the CHARISMA trial. Accordingly, as the directionality of future anti-thrombotic therapy, in addition to the creation of a drug capable of achieving further high event inhibition ratio, it is considered that an multidisciplinary therapy of the combination use of superior drugs may be groped with considering not only drug effect strength but also hemorrhagic side effects and carrying out setting of appropriate usage and dose of such drugs.

Aspirin is an irreversible inhibitor of a rate-limiting enzyme of the arachidonic acid metabolic pathway, cyclooxygenase-1 (COX-1). The COX has subtypes and in addition to the constitutive type COX-1, there is known an inducible type COX-2 which is expressed at the time of inflammation. According to the latest review, it has been reported that production of thromboxane A2 (TXA₂) which causes having strong platelet aggregation activity and vasoconstrictive activity is originated from platelet COX-1, and on the other hand, production of prostacyclin (PGI₂) which causes strong platelet aggregation inhibitrory activity and vasodilative activity is originated mainly from vascular endothelial COX-2 and partly from COX-1 (J. Clin. Invest., 116: 4 - 15, 2006). In addition, there is a study report that the gastric ulcer caused by aspirin and NSAIDs is caused by inhibiting both of COX-1 and COX-2 (Gastroenterology, 119: 796 - 14, 2000).

On the other hand, it is known that COX-1 selective inhibitors do not generate the gastrointestinal side effects which have been found in the conventional NSAIDs (Patent Reference 1). However, there are no reports stating that these compounds enhance anti-thrombotic action of other anti-thrombotic agents and gastrointestinal disorders and the like side effects are not generated by their concomitant use with other anti-thrombotic agents.

Patent Reference 1: International publication No. WO 03/040110
Patent Reference 2: Specification of U.S. Patent No. 4, 529, 596
Patent Reference 3: Specification of U.S. Patent No. 4, 847, 265
Non-patent Reference 1: Lancet 348: 1329-99, 1996
Non-patent Reference 2: Am. Heart J., 145: 595-601, 2003
Non-patent Reference 3: N.Eng.J.Med.,354: 1706-17, 2006

### DISCLOSURE OF THE INVENTION

### PROBLEMS THAT THE INVENTION IS TO SOLVE

An object of the present invention is to provide an excellent pharmaceutical composition for preventing and/or treating vascular diseases, which shows enhanced effectiveness and fewer side effects in comparison with the aspirin, clopidogrel and the like drugs put on the market for vascular diseases and their combination use therapy.

### MEANS FOR SOLVING THE PROBLEMS

The present inventors have confirmed that when a "COX-1 selective inhibitor or a pharmaceutically acceptable salt thereof" (e.g., 3-methoxy-1,5-bis(4-methoxyphenyl)-1H-1,2,4-triazole (Compound A, hereinafter)) and an anti-thrombotic agent having different action mechanism, methyl (+)-(S)-2-(2-chlorophenyl)-2-(4,5,6,7-tetrahydrothieno[3,2-c]pyridin-5-yl)acetate (clopidogrel, hereinafter) or a pharmaceutically acceptable salt thereof are concomitantly used, it shows markedly excellent effect for preventing and/or treating vascular diseases and further more, side effects such as gastrointestinal disorders and the like is not generated, in comparison with a case of administering Compound A or clopidogrel alone and a case of concomitantly using aspirin and clopidogrel, and thereby have accomplished the present invention.

An object of the present invention is to provide an agent for preventing and/or treating vascular diseases, **characterized in that** a COX-1 selective inhibitor or a pharmaceutically acceptable salt thereof is combined with clopidogrel or a pharmaceutically acceptable salt thereof.
Another object of the present invention is to provide a pharmaceutical composition, which comprises 1) a COX-1 selective inhibitor or a pharmaceutically acceptable salt thereof and 2) clopidogrel or a pharmaceutically acceptable salt thereof.
A further object of the present invention is to provide a pharmaceutical composition for preventing and/or treating vascular diseases, which comprises 1) a COX-1 selective inhibitor or a pharmaceutically acceptable salt thereof and 2) clopidogrel or a pharmaceutically acceptable salt thereof.
A further object of the present invention is to provide use of a COX-1 selective inhibitor or a pharmaceutically acceptable salt thereof for the manufacture of a medicament for preventing and/or treating vascular diseases in combination with clopidogrel or a pharmaceutically acceptable salt thereof.
A further obj ect of the present invention is to provide a method for preventing and/or treating vascular diseases, which comprises administering 1) an effective amount of clopidogrel or a pharmaceutically acceptable salt thereof and 2) an effective amount of a COX-1 selective inhibitor or a pharmaceutically acceptable salt thereof to the aforementioned human or animal.
A further object of the present invention is to provide a process for producing a pharmaceutical composition for preventing and/or treating vascular diseases, which comprises mixing 1) a COX-1 selective inhibitor or a pharmaceutically acceptable salt thereof, and 2) clopidogrel or a pharmaceutically acceptable salt thereof, and a pharmaceutically acceptable excipient.
A further object of the present invention is to provide a pharmaceutical composition for preventing and/or treating vascular diseases, which comprises 1) a formulation comprising a COX-1 selective inhibitor or a pharmaceutically acceptable salt thereof as an active ingredient and 2) a package insert indicating that said formulation is used in combination with a formulation containing clopidogrel or a pharmaceutically acceptable salt thereof as an active ingredient.

### EFFECT OF THE INVENTION

The present invention is useful for providing a pharmaceutical composition for preventing and/or treating vascular diseases. Further, the present invention is particularly useful for providing a pharmaceutical composition for preventing and/or treating the above-mentioned diseases, in which side effects such as gastrointestinal disorders and the like is reduced.

### BRIEF DESCRIPTION OF THE DRAWINGS

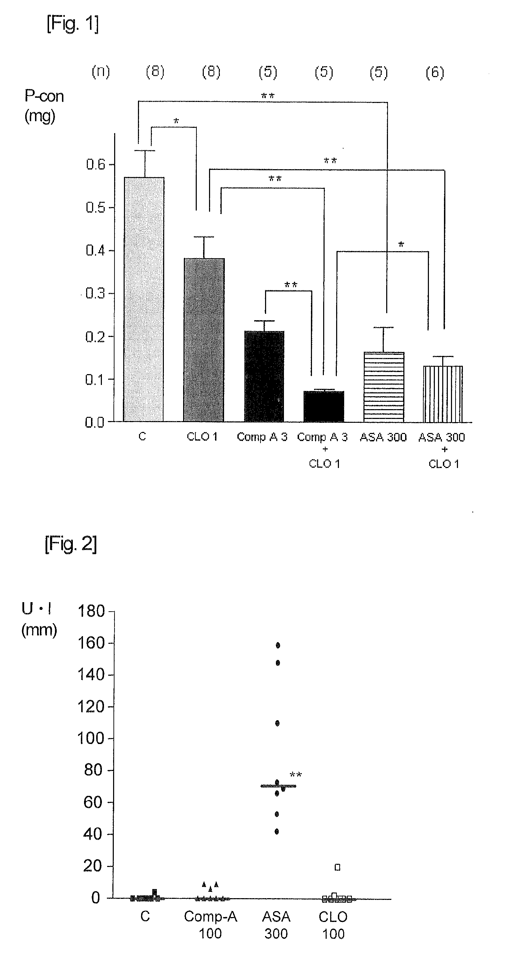

[Fig. 1] Fig. 1 shows the amount of thrombi in a guinea pig iron chloride-induced thrombus model, in the case of administering Compound A, aspirin or clopidogrel alone, in the case of concomitantly administering Compound A or aspirin with clopidogrel or in the case of administering the vehicle alone. P-con of the vertical axis represents total protein of thrombi. On the horizontal axis, C represents a vehicle administration group (control), and CLO 1 represents a clopidogrel 1 mg/kg administration group, Comp A3 represents a Compound A 3 mg/kg administration group, Comp A3 + CLO 1 represents a concomitant administration group of 3 mg/kg of Compound A and 1 mg/kg of clopidogrel, ASA 300 represents an aspirin 300 mg/kg administration group, and ASA 300 + CLO 1 represents a concomitant administration group of 300 mg/kg of aspirin and 1 mg/kg of clopidogrel, respectively. The * in the table indicates that it is a group having a significant difference with a critical rate of less than 5%, as a result of Student's t-test. The ** in the table indicates that it is a group having a significant difference with a critical rate of less than 1%, as a result of Student's t-test. The numeral (n) in the parentheses represents the number of animals of guinea pigs in each group.

[Fig. 2] Fig. 2 shows the length of gastric mucosal lesion in the case of administering Compound A, aspirin or clopidogrel alone to normal guinea pigs. U·I (mm) of the vertical axis represents the sum total of lengths of lesions found on the gastric mucosa surface, and the result is plotted for each individual. In addition, the horizontal line in the drawing represents median value of each group. On the horizontal axis, C represents a vehicle administration group (control), and CLO 1 represents a clopidogrel 1 mg/kg administration group, Comp-A 100 represents a Compound-A 100 mg/kg administration group, ASA 300 represents an aspirin 300 mg/kg administration group, and CLO 100 represents a clopidogrel 100 mg/kg administration group, respectively. The ** in the figure indicates that it is a group having a significant difference with a critical rate of less than 1 %, as a result of Wilcoxon rank sum test.

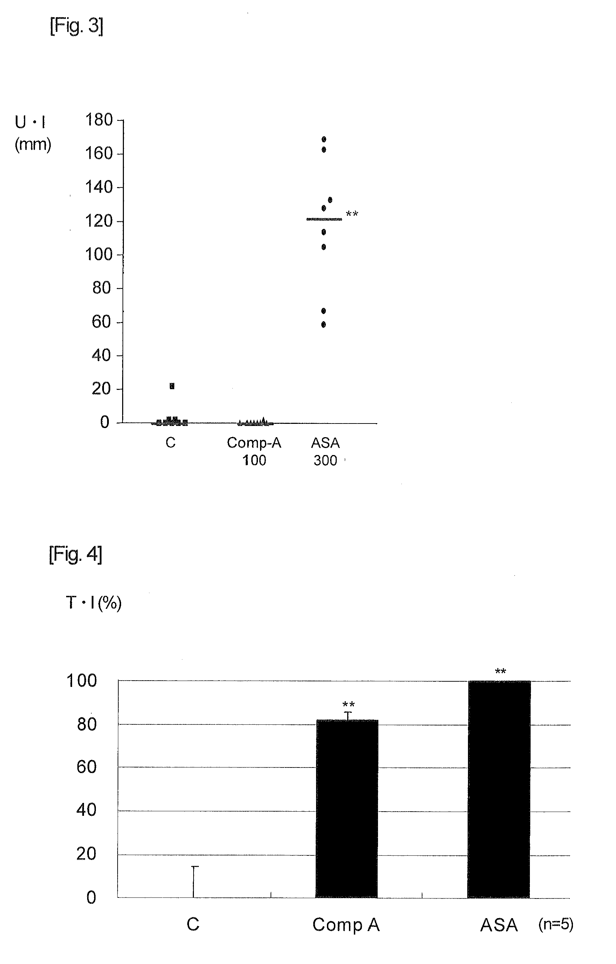

[Fig. 3] Fig. 3 shows the length of gastric mucosal lesion in the case of concomitantly administering Compound A or aspirin together with clopidogrel to normal guinea pigs. U•I (mm) of the vertical axis represents the sum total of lengths of lesions found on the gastric mucosa surface, and the result is plotted for each individual. In addition, the horizontal line in the drawing represents median value of each group. On the horizontal axis, C represents a concomitant administration group of vehicle and 3 mg/kg of clopidogrel, and Comp-A 100 represents a concomitant administration group of 100 mg/kg of Compound-A and 3 mg/kg of clopidogrel, and ASA 300 represents a concomitant administration group of 300 mg/kg of aspirin and 3 mg/kg of clopidogrel, respectively. The ** in the figure indicates that it is a group having a significant difference with a critical rate of less than 1%, as a result of Wilcoxon rank sum test.

[Fig. 4] Fig. 4 is a graph showing inhibition ratio of aggregation-induced TXB₂ production using guinea pig whole blood when Compound A or aspirin was concomitantly administered with clopidogrel. On the horizontal axis, C represents a vehicle administration group (control), and Comp A represents a concomitant administration group of 3 mg/kg of Compound A and 1 mg/kg of clopidogrel, and ASA represents a concomitant administration group of 300 mg/kg of aspirin and 1 mg/kg of clopidogrel, respectively. T•I (%) of the vertical axis represents inhibition ratio when the vehicle administration group was regarded as inhibition ratio 0%. The ** in the table indicates that it is a group having a significant difference with a critical rate of less than 1%, as a result of Student's t-test. The numeral in the parentheses represents the number of animals of guinea pigs in each group.

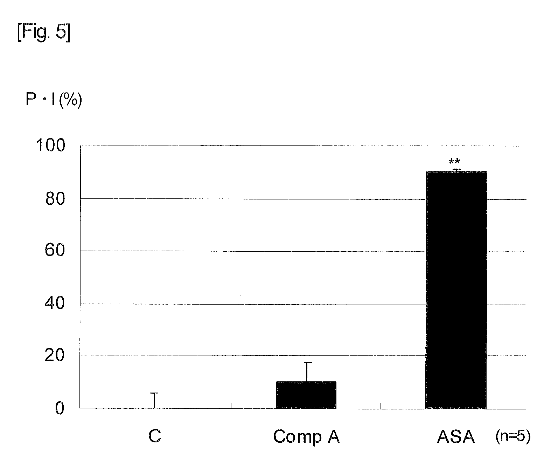

[Fig. 5] Fig. 5 is a graph showing inhibition ratio of LPS-induced PGE₂ production using guinea pig whole blood when Compound A or aspirin was concomitantly administered with clopidogrel. On the horizontal axis, C represents a vehicle administration group (control), and Comp A represents a concomitant administration group of 3 mg/kg of Compound A and 1 mg/kg of clopidogrel, and ASA represents a concomitant administration group of 300 mg/kg of aspirin and 1 mg/kg of clopidogrel, respectively. P•I (%) of the vertical axis represents inhibition ratio when the vehicle administration group was regarded as inhibition ratio 0%. The** in the table indicates that it is a group having a significant difference with a critical rate of less than 1%, as a result of Student's t-test. The numeral in the parentheses represents the number of animals of guinea pigs in each group.

### BEST MODE FOR CARRYING OUT THE INVENTION

The following shows preferable embodiments of the present invention.
(1) An agent for preventing and/or treating vascular diseases, **characterized in that** Compound A or a pharmaceutically acceptable salt thereof is combined with clopidogrel or a pharmaceutically acceptable salt thereof.

(2) An agent for preventing and/or treating arterial thrombosis, ischemic heart disease [e.g., angina pectoris (e.g., stable angina pectoris, unstable angina pectoris including impending infarction, and the like), myocardial infarction (e.g., acute myocardial infarction and the like), coronary thrombosis and the like], ischemic brain disease [e.g., cerebral infarction (e.g., acute cerebral thrombosis and the like), cerebral thrombosis (e.g., cerebral embolism and the like), transient cerebral ischemia (e.g., transient ischemic attack and the like) and the like], pulmonary embolism, peripheral circulation disorder [e.g., thromboangiitis obliterans (namely Buerger disease), Raynaud disease and the like], restenosis and reocclusion [e.g., restenosis and/or reocclusion after percutaneous transluminal coronary angioplasty (PTCA), restenosis and reocclusion after administration of a thrombolytic agent (e.g., tissue plasminogen activation factor (tPA) or the like)] or essential thrombocytosis, **characterized in that** Compound A or a pharmaceutically acceptable salt thereof is combined with clopidogrel or a pharmaceutically acceptable salt thereof.

(3) An agent for preventing and/or treating arterial thrombosis, ischemic heart disease [e.g., angina pectoris (e.g., stable angina pectoris, unstable angina pectoris including impending infarction, and the like), myocardial infarction (e.g., acute myocardial infarction and the like), coronary thrombosis and the like], ischemic brain disease [e.g., cerebral infarction (e.g., acute cerebral thrombosis and the like), cerebral thrombosis (e.g., cerebral embolism and the like), transient cerebral ischemia (e.g., transient ischemic attack and the like) and the like] or restenosis and reocclusion [e.g., restenosis and/or reocclusion after percutaneous transluminal coronary angioplasty (PTCA), restenosis and reocclusion after administration of a thrombolytic agent (e.g., tissue plasminogen activation factor (tPA) or the like)], **characterized in that** Compound A or a pharmaceutically acceptable salt thereof is combined with clopidogrel or a pharmaceutically acceptable salt thereof.

The following illustratively describes suitable examples of various definitions included in the scope of the present invention, described in the above and following of this specification.

The Compound A to be used in the present invention is a compound represented by the following structural formula (I).

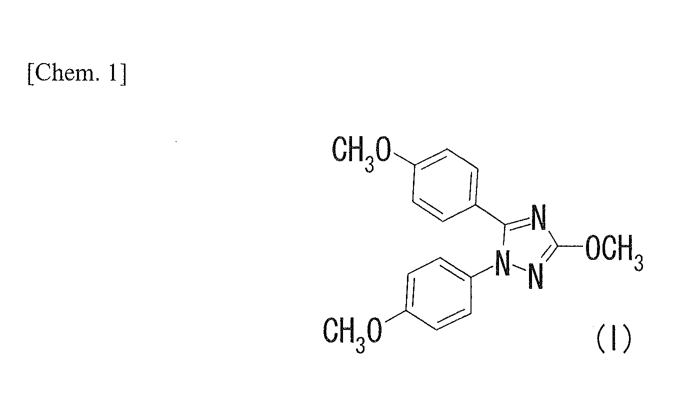

The clopidogrel to be used in the present invention is a compound represented by the following structural formula (II).

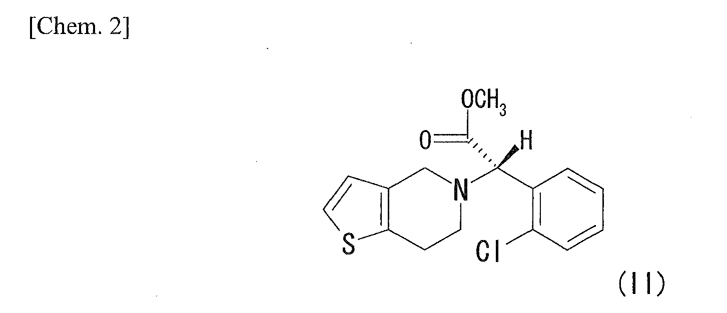

The "vascular disease" means a disease or symptom caused by thrombi in the blood vessel. Illustratively, it includes arterial thrombosis, ischemic heart disease [e.g., angina pectoris (e.g., stable angina pectoris, unstable angina pectoris including impending infarction, and the like), myocardial infarction (e.g., acute myocardial infarction and the like), coronary thrombosis and the like], ischemic brain disease [e.g., cerebral infarction (e.g., acute cerebral thrombosis and the like), cerebral thrombosis (e.g., cerebral embolism and the like), transient cerebral ischemia (e.g., transient ischemic attack and the like) and the like], pulmonary embolism, peripheral circulation disorder [e.g., thromboangiitis obliterans (namely Buerger disease), Raynaud disease and the like], restenosis and reocclusion [e.g., restenosis and/or reocclusion after percutaneous transluminal coronary angioplasty (PTCA), restenosis and reocclusion after administration of a thrombolytic agent (e.g., tissue plasminogen activation factor (tPA) or the like)], essential thrombocytosis and the like, though not limited thereto.

According to this specification, the "COX-1 selective inhibitor" means a substance having a property in that its inhibitory activity for COX-1 is stronger than its inhibitory activity for COX-2. Preferably, it means "a compound having an aggregation-induced Thromboxane B₂ production inhibition ratio of 70% or more based on the vehicle group and also having an LPS-induced Prostaglandin E2 (PGE₂) production inhibition ratio of less than 20% based on the vehicle group, at the time of administering effective amount of a drug to a guinea pig iron chloride-induced thrombus model in which clopidogrel is concomitantly used". Illustratively, Compound A for example is included.

The "compound having an aggregation-induced Thromboxane B₂ production inhibition ratio of 70% or more based on the vehicle group and also having an LPS-induced Prostaglandin E2 (PGE₂) production inhibition ratio of less than 20% based on the vehicle group, at the time of administering effective amount of a drug to a guinea pig iron chloride-induced thrombus model in which clopidogrel is concomitantly used" means a compound having an aggregation-induced Thromboxane B₂ production inhibition ratio of 70% or more based on the vehicle group and also having an LPS-induced Prostaglandin E2 (PGE2) production inhibition ratio of less than 20% based on the vehicle group, calculated by the method described in Example 3 of this application.

As the "agent for preventing and/or treating vascular diseases, **characterized in that** a COX-1 selective inhibitor or a pharmaceutically acceptable salt thereof is combined with clopidogrel or a pharmaceutically acceptable salt thereof" of the present invention, it includes a pharmaceutical composition (mixed preparation) for preventing and/or treating vascular diseases, which comprise an effective amount of a COX-1 selective inhibitor or a pharmaceutically acceptable salt thereof and an effective amount of clopidogrel or a pharmaceutically acceptable salt thereof, and a kit which contains two kinds of preparations, namely, as a first formulation, an agent for preventing and/or treating vascular diseases, comprises a COX-1 selective inhibitor or a pharmaceutically acceptable salt thereof as an active ingredient and, as a second formulation, an agent for preventing and/or treating vascular diseases, comprises clopidogrel or a pharmaceutically acceptable salt thereof as an active ingredient. In this case, the two kinds of preparations are administered simultaneously or separately through the same or different route of administration.

The above-mentioned "kit which contains two kinds of preparations" contains two kinds of formulations containing respective active ingredients in such a combination that it can be used in the combination use therapy of these active ingredients, it is exemplified that a packed product which may contain, as occasion demands, an additional formulation and a display member, such as a placebo preparation and the like, that facilitates the administration in response to the respective administration periods. Also, the "simultaneously" means that the first preparation and second preparation are administered at the same time through the same route of administration, and the "separately" means that the first preparation and second preparation are administered through the same or different route of administration at the same or different administration frequency or administration interval. Preferably, by taking bioavailability, stability and the like of respective formulations into consideration, these are administered simultaneously or separately under administration conditions such as formulation prescription, route of administration or administration frequency and the like suited for the respective formulations.

The Compound A and/or or a pharmaceutically acceptable salt thereof can be easily obtained by the production methods described in Patent Reference 1 or modified production methods thereof.

Clopidogrel or a pharmaceutically acceptable salt thereof can be easily obtained by the production methods described in U.S. Patent No. 4, 529, 596 or U.S. Patent No. 4, 847, 265 or modified production methods thereof.

The compound having an aggregation-induced Thromboxane B₂ production inhibition ratio of 70% or more based on the vehicle group and also having an LPS-induced Prostaglandin E2 (PGE₂) production inhibition ratio of less than 20% based on the vehicle group, at the time of administering effective amount of a drug to a guinea pig iron chloride-induced thrombosis model in which clopidogrel is concomitantly used, or a pharmaceutically acceptable salt thereof, can be easily obtained by evaluating the compounds which can be obtained by the embodiments of conventional technology at the filing of this application, by the method of Example 3.

Suitable salts of the COX-1 selective inhibitor are generally used nontoxic salts which are acceptable as medicines, for example, metal salts such as alkali metal salts (e.g., sodium salt, potassium salt and the like) or alkaline earth metal salts (e.g., calcium salt, magnesium salt and the like), ammonium salt, organic base salts (e.g., trimethylammonium salt, triethylammonium salt, pyridinium salt, picoline salt, dicyclohexylammnonium salt and the like), organic acid salts (e.g., acetate, maleate, tartarate, methanesulfonate, benzenesulfonate, formate, toluenesulfonate, trifluoroacetate and the like), inorganic acid salts (e.g., hydrochloride, hydrobromide, sulfate, phosphate and the like), salts with amino acids (e.g., arginine, aspartic acid, glutamic acid and the like), and the like are exemplified. Suitable salts of clopidogrel are generally used non-toxic salts which are acceptable as medicines, for example, organic acid salts (e.g., acetate, malonate, tartarate, methanesulfonate, benzenesulfonate, formate, toluenesulfonate, trifluoroacetate and the like), inorganic acid salts (e.g., hydrochloride, hydrobromide, sulfate, phosphate and the like), amino acid salts (e.g., alginate, aspartate, glutamate and the like), and the like are exemplified. Particularly preferred is sulfate.

The COX-1 selective inhibitor and clopidogrel or pharmaceutically acceptable salts thereof can also form hydrates or pharmaceutically acceptable various solvates. These hydrates and solvates are also included in the present invention.

The pharmaceutical composition for preventing and/or treating vascular diseases, **characterized in that** the COX-1 selective inhibitor or a pharmaceutically acceptable salt thereof is combined with clopidogrel or a pharmaceutically acceptable salt thereof in the present invention, can be produced by preparing as a mixed preparation or separate preparations from an effective amount of a COX-1 selective inhibitor or a pharmaceutically acceptable salt thereof and an effective amount of clopidogrel or a pharmaceutically acceptable salt thereof, by a generally used method using medicinal carrier, filler and the like which are generally used in said field. These pharmaceutical preparations can be prepared by a generally used method using medicinal carrier, filler and the like which are generally used in said field. The administration may be any form of oral administration by tablets, pills, capsules, granules, powders, solutions and the like, or parenteral administration by intraarticular, intravenous, intramuscular and the like injections, suppositories, eye drops, eye ointments, percutaneous solutions, ointments, percutaneous patches, transmucosal solutions, transmucosal patches, inhalations and the like.

As the solid composition for oral administration by the present invention, tablets, powders, granules and the like are used. In such a solid composition, one or two or more active ingredients are mixed with at least one inert diluent such as lactose, mannitol, glucose, hydroxypropyl-cellulose, microcrystalline cellulose, starch, polyvinyl pyrrolidone and/or magnesium aluminometasilicate, and the like. In accordance with the conventional procedure, the composition may contain additive agents other than the inert diluent, for example, lubricants such as magnesium stearate and the like, disintegrating agents such as calcium cellulose glycolate and the like, stabilizers or solubilizing agents. As occasion demands, the tablets or pills may be coated with a sugar coating or a film of gastric or enteric substance, such as of sucrose, gelatin, hydroxypropylcellulose, hydroxypropylmethyl-cellulose phthalate and the like.

The liquid composition for oral administration includes pharmaceutically acceptable emulsions, solutions, suspensions, syrups or elixirs and the like and contains a generally used inert diluent such as purified water or ethanol. In addition to the inert diluent, said liquid composition may contain auxiliaries such as solubilizing agents, moistening agents or suspending agents and the like, sweeteners, flavors, aromatics and antiseptics.

The injections for parenteral administration include aseptic aqueous or nonaqueous solutions, suspensions or emulsions. As the aqueous solutions or suspensions, for example, distilled water for injection or physiological saline is included. As the nonaqueous solutions or suspensions, for example, plant oil such as propylene glycol, polyethylene glycol or olive oil or the like, alcohols such as ethanol or the like, polysorbate 80 (official name) and the like. Such a composition may further contain a tonicity agent, an antiseptic, a moistening agent, an emulsifying agent, a dispersing agent, a stabilizing agent or a solubilization assisting agent. These are sterilized by filtration through a bacteria retaining filter, blending of a germicide or irradiation. Alternatively, they may be used by firstly making into sterile solid compositions and dissolving or suspending them in sterile water or a sterile solvent for injection prior to their use.

The transmucosal preparations such as transnasal agents and the like are used in a liquid or semi-liquid form and can be produced in accordance with a conventionally known method. For example, conventionally known pH adjusting agents, antiseptics, thickeners and fillers are optionally added and formed into a liquid or semi-solid form. The transnasal agents are administered using a general sprayer, nasal drop container, tube, nasal cavity insertion tool or the like.

1) The agent which contains COX-1 selective inhibitor or a pharmaceutically acceptable salt thereof as an active ingredient or 2) the agent which contains COX-1 selective inhibitor or a pharmaceutically acceptable salt thereof and clopidogrel or a pharmaceutically acceptable salt thereof as an active ingredients, both to be used in the present invention, are administered to patients having vascular diseases, and suitable daily dose of the 1) COX-1 selective inhibitor or a pharmaceutically acceptable salt thereof or 2) COX-1 selective inhibitor or a pharmaceutically acceptable salt thereof and clopidogrel or a pharmaceutically acceptable salt thereof is generally from about 0.001 to 100 mg/kg per body weight in the case of oral administration, which is administered once a day or by dividing it into 2 to 4 portions. In the case of intravenous administration, the daily dose is from about 0.0001 to 10 mg/kg per body weight is suitable and administered once a day or by dividing it into two or more portions. In addition, in the case of the transmucosal agents, from about 0.001 to 100 mg/kg per body weight is administered once a day or by dividing it into two or more portions. The dose is optionally determined in response to individual cases by taking symptoms, ages, sexes and the like into consideration.

### EXAMPLES

The object of the following Examples is to describe the present invention further illustratively, and the present invention is not limited to the following Examples. Though the present invention is sufficiently described by Examples, it can be understood by those skilled in the art that there will be various alterations and modifications. Thus, such alterations and modifications are included in the present invention without departing from the scope of the present invention.

### Example 1

### (Experiment)

Verification of anti-thrombotic action was carried out using a ferric chloride-induced thrombosis model in guinea pigs, by partially modifying the experiment described in "Thrombosis Research" (1990, vol. 60, p. 269 - 280). Using 0.5% methyl cellulose solution as the vehicle, clopidogrel solution, aspirin suspension and Compound A suspension were prepared. The clopidogrel solution was orally administered 2 hours before the thrombus induction, and the aspirin suspension and Compound A suspension were orally administered 1 hour before the thrombus induction, to male Hartley guinea pigs which had been subjected to fasting. Thrombus was induced by the following procedure. Each guinea pig was laparotomized while under pentobarbital anesthesia, and abdominal aorta was carefully detached from the surrounding tissues. A paraffin film was spread under the detached blood vessel and a 5 mm x 4 mm piece of filter paper instilled with 10% FeCl₃ solution was put on the blood vessel surface, which was protected from light. The filter paper was removed 10 minutes thereafter, followed by 45 minutes of standing under subdued light. Both ends of the injured part of the detached blood vessel were closed with clips, and inside thereof was cut out using scissors to collect the blood vessel. By cutting open the thus collected blood vessel vertically, the thrombi formed in the blood vessel were taken out using a pair of tweezers and dissolved in 0.5 mol/l of NaOH. Protein concentration was determined using DC protein assay kit (mfd. by BIO-RAD Laboratories) and in accordance with the protocols. Using total protein content of the formed thrombi as the index of anti-thrombotic effect, Statistical analysis between respective groups was performed by using Student's t-test.

### (Results)

Measured results of total protein content of thrombi are shown in Fig. 1. In comparison with the control (vehicle administration group) (C), clopidogrel 1 mg/kg administration group (CLO 1), Compound A 3 mg/kg administration group (Comp A3) and aspirin 300 mg/kg administration group (ASA 300) showed a statistically significant difference. In addition, a group in which 1 mg/kg of clopidogrel and 3 mg/kg of Compound A were concomitantly administered (Comp A3 + CLO 1) showed a statistically significant difference from the clopidogrel single administration group (CLO 1) and Compound A single administration group (Comp A 3). On the other hand, a group in which 1 mg/kg of clopidogrel and 300 mg/kg of aspirin were concomitantly administrated (ASA 300 + CLO 1) showed a significant difference from the clopidogrel single administration group (CLO 1), but a significant difference was not found in comparison with the aspirin single administration group (ASA 300), so that distinct synergistic effect was not observed. In addition, a concomitant administration group of clopidogrel and Compound A (Comp A3 + CLO 1) showed a statistically significant difference from a concomitant administration group of clopidogrel and aspirin (ASA 300 + CLO 1). That is, it was shown that the Compound A considerably enhanced anti-thrombotic effect of clopidogrel compared to the case of using aspirin at the dose in which it shows anti-thrombotic effect equal to or larger than Compound A (100 times of Compound A). Accordingly, it was shown that Compound A has the effect to enhance anti-thrombotic efficacy of clopidogrel, which is far superior to the conventionally used aspirin.

### Example 2

### (Experiment)

Examination of the influence of drugs exerting upon gastric mucosa was carried out using normal guinea pigs. Using 0.5% methyl cellulose solution as the vehicle, clopidogrel solution, aspirin suspension and Compound A suspension were prepared. The clopidogrel solution, aspirin suspension or Compound A suspension was orally administered by gavage to male Hartley guinea pigs which had been subjected to fasting. Regarding the dose of each drug in the case of single drug evaluation, aspirin was administered at its pharmacologically effective dose, 300 mg/kg, and Compound A or clopidogrel at 100 mg/kg which is a dose of about 30 times higher than its pharmacologically effective dose. Also, in the evaluation at the time of concomitant administration of clopidogrel with aspirin or Compound A, in addition to 3 mg/kg of clopidogrel, 300 mg/kg of aspirin was simultaneously administered in the aspirin concomitant use group, and 100 mg/kg of Compound A in the Compound A concomitant use group. After 3 hours of the administration, each guinea pig was sacrificed by deep anesthesia with carbon dioxide, and the stomach was quickly removed. The gullet part of the extracted stomach was ligated, 15 ml of 4% neutral buffered formalin solution was injected from the pylorus part, and then light fixation was carried out by immersing in the same solution for about 1 hour after ligating the pylorus part. Thereafter, the stomach was cut open along the greater curvature of stomach, and the length (mm) of gastric mucosal lesion was measured using a stereoscopic microscope. Statistical analysis between respective groups was performed by using Wilcoxon rank sum test.

### (Results)

Measured results of the gastric mucosal lesion are shown in Fig. 2 and Fig. 3. Gastric mucosal damage was clearly formed in the group of aspirin (ASA) 300 mg/kg administration. On the other hand, a clear action for gastric mucosa cannot be confirmed in the Compound A (Comp A) and clopidogrel (CLO) 100 mg/kg administration groups. Gastric mucosal damage was also clearly induced by aspirin 300 mg/kg in the case of clopidogrel 3 mg/kg concomitant administration. As for aspirin, the tendency was shown that gastric mucosal damage at the time of its concomitant administration with clopidogrel becomes worse than the gastric mucosal damage which is formed when aspirin alone is used. On the other hand, a clear action for gastric mucosa cannot be confirmed in the group of concomitant use administration of Compound A (Comp A) 100 mg/kg and 3 mg/kg clopidogrel.

### Example 3

### (Experiment)

Examination on the selectivity of inhibitory effect on Cyclooxygenase (COX)-1/2 was carried out based on, as indexes, the coagulation-induced Thromboxane B₂ (TXB₂) production inhibition (COX-1 inhibition) and the LPS-induced Prostaglandin E₂ (PGE₂) production inhibition using guinea pig whole blood. As the vehicle, 0.5% methyl cellulose solution was used. By dissolving clopidogrel and suspending aspirin and Compound A therein, clopidogrel was orally administered 2 hours before the blood collection, and aspirin and Compound A 1 hour before thereof, to male Hartley guinea pigs which had been subjected to fasting (drug administered groups). On the other hand, as the vehicle administration group, clopidogrel was orally administered 2 hours before the blood collection, and the vehicle 1 hour before thereof. Each guinea pig was laparotomized while under ether anesthesia, 4 ml of blood was collected from the abdominal aorta, and 1 ml thereof was put into an anticoagulant-un-added tube and allowed to stand still and then 3 ml thereof was put into a tube charged with 300 µl of sodium citrate, followed by tipping mixing. The anticoagulant-un-added whole blood was incubated at 37°C for 1 hour and then indometacin was added to a final concentration of 10 µM, followed by centrifugation at 15000 rpm and at 4°C to collect serum. TXB₂ concentration in the serum was measured using a TXB₂ EIA Kit (mfd. by Cayman Chemicals). The sodium citrate-added whole blood was mixed with LPS to a final concentration of 100 µg/ml, incubated at 37°C for 24 hours, mixed with indometacin to a final concentration of 10 µM and then centrifuged at 15000 rpm and at 4°C to collect plasma. A 100 µl portion of the collected plasma was mixed with 400 µl of methanol and centrifuged at 15000 rpm and at 4°C, and then entire volume of the supernatant was put into a glass tube and evaporated to dryness using an evaporator. By adding 100 µl of EIA buffer, the dried residue in the glass tube was completely dissolved. PGE₂ concentration in the buffer was measured using a PGE₂ EIA Kit (mfd. by Cayman Chemicals). Inhibitory rate of each of the TXB₂ concentration and PGE₂ concentration relative to the vehicle group was calculated using a calculation formula [100 - (drug administration group concentration/solvent administration concentration) x 100 (%)], and significant difference from the vehicle group was tested using Student's t-test.

### (Results)

Results of coagulation-induced TXB₂ production inhibition are shown in Fig. 4. Under concomitant use with 1 mg/kg of clopidogrel, both of the 3 mg/kg of Compound A (Comp A) and 300 mg/kg of aspirin (ASA) showed statistically significant inhibitory effects on coagulation-induced TXB₂ production in comparison with the vehicle administration group (C), and the inhibitory rates were 82.1 % and 100.0%, respectively. Results of LPS-induced PGE₂ production inhibition are shown in Fig. 5. Significant inhibitory effect on PGE₂ production in comparison with the vehicle administration group was not confirmed by the concomitant administration of 1 mg/kg of clopidogrel and 3 mg/kg of Compound A. On the other hand, it was able to confirm significant PGE₂ production inhibition in comparison with the solvent administration group, when 1 mg/kg of clopidogrel and 300 mg/kg of aspirin were concomitantly administrated. The inhibitory rates were 10.1 % and 90.4%, respectively.

### INDUSTRIAL APPLICABILITY

The pharmaceutical composition of the present invention is useful as a pharmaceutical composition for preventing and/or treating vascular diseases is provided. Further, the pharmaceutical composition of the present invention is particularly useful as a pharmaceutical composition for preventing and/or treating the above-mentioned diseases, in which gastrointestinal disorders and the like side effects were reduced is provided. Furthermore, the pharmaceutical composition of the present invention is particularly useful as a pharmaceutical composition for preventing and/or treating arterial thrombosis, ischemic heart disease [e.g., angina pectoris (e.g., stable angina pectoris, unstable angina pectoris including impending infarction, and the like), myocardial infarction (e.g., acute myocardial infarction and the like), coronary thrombosis and the like], ischemic brain disease [e.g., cerebral infarction (e.g., acute cerebral thrombosis and the like), cerebral thrombosis (e.g., cerebral embolism and the like), transient cerebral ischemia (e.g., transient ischemic attack and the like) and the like], pulmonary embolism, peripheral circulation disorder [e.g., thromboangiitis obliterans (namely Buerger disease), Raynaud disease and the like], restenosis and reocclusion [e.g., restenosis and/or reocclusion after percutaneous transluminal coronary angioplasty (PTCA), restenosis and reocclusion after administration of a thrombolytic agent (e.g., tissue plasminogen activation factor (tPA) or the like)] or essential thrombocytosis, is provided.

## Claims

1. An agent for preventing and/or treating vascular diseases, **characterized in that** a COX-1 selective inhibitor or a pharmaceutically acceptable salt thereof is combined with clopidogrel or a pharmaceutically acceptable salt thereof.

2. A pharmaceutical composition, which comprises 1) a COX-1 selective inhibitor or a pharmaceutically acceptable salt thereof and 2) clopidogrel or a pharmaceutically acceptable salt thereof.

3. The pharmaceutical composition described in claim 2, wherein the COX-1 selective inhibitor is 3-methoxy-1,5-bis(4-methoxyphenyl)-1H-1,2,4-triazole or a pharmaceutically acceptable salt thereof.

4. A pharmaceutical composition for preventing and/or treating vascular diseases, which comprises 1) a COX-1 selective inhibitor or a pharmaceutically acceptable salt thereof and 2) clopidogrel or a pharmaceutically acceptable salt thereof.

5. The pharmaceutical composition described in claim 4, wherein the COX-1 selective inhibitor is 3-methoxy-1,5-bis(4-methoxyphenyl)-1H-1,2,4-triazole or a pharmaceutically acceptable salt thereof.

6. Use of a COX-1 selective inhibitor or a pharmaceutically acceptable salt thereof for the manufacture of a medicament for preventing and/or treating vascular diseases in combination with clopidogrel or a pharmaceutically acceptable salt thereof.

7. The use described in claim 6, wherein the COX-1 selective inhibitor is 3-methoxy-1,5-bis(4-methoxyphenyl)-1H-1,2,4-triazole or a pharmaceutically acceptable salt thereof.

8. A method for preventing and/or treating vascular diseases, which comprises administering 1) an effective amount of clopidogrel or a pharmaceutically acceptable salt thereof and 2) an effective amount of a COX-1 selective inhibitor or a pharmaceutically acceptable salt thereof to the aforementioned human or animal.

9. The method described in claim 8, wherein the COX-1 selective inhibitor is 3-methoxy-1,5-bis(4-methoxyphenyl)-1H-1,2,4-triazole or a pharmaceutically acceptable salt thereof.

10. A pharmaceutical composition for preventing and/or treating vascular diseases, which comprises 1) a formulation comprising a COX-1 selective inhibitor or a pharmaceutically acceptable salt thereof as an active ingredient and 2) a package insert indicating that said formulation is used in combination with a formulation containing clopidogrel or a pharmaceutically acceptable salt thereof as an active ingredient.
